# EUROPEAN PATENT SPECIFICATION

(11) **EP 0 759 736 B1**
(45) Date of publication and mention of the grant of the patent: **28.10.1998**
(21) Application number: 95918681.8
(22) Date of filing: 16.05.1995
(51) Int. Cl.: A61K 7/00, A61K 9/127, A61K 7/50

(54) **LIPOSOME FORMING COMPOSITIONS**
LIPOSOMBILDENDE ZUSAMMENSETZUNGEN
COMPOSITIONS FORMANT DES LIPOSOMES

(30) Priority: 16.05.1994 GB 9409763
(43) Date of publication of application: 05.03.1997
(73) Proprietor: PHARES PHARMACEUTICAL RESEARCH N.V., Curacao Netherlands Antilles (AN)
(72) Inventor: LEIGH, Steven Stevens, Hewlett & Perkins, London EC4Y 1LL (GB)
(74) Representative: Gaunt, Robert John
(86) International application number: GB9501096
(87) International publication number: WO9531172

(56) References cited:
- EP-A- 0 130 577
- EP-A- 0 158 441
- EP-A- 0 557 825
- DE-A- 4 021 083
- FR-A- 2 550 706
- FR-A- 2 686 509
- DEUTSCHE APOTHEKER ZEITUNG, vol. 41, no. 14, 14 October 1993 pages 43-50, NÜRNBERG ET AL. 'OLEOBALNEOLOGIKA, Verteilungszustände lipophile Stoffe in Badeflotte'

## Description

This invention relates to oil-containing compositions which form liposomes or vesicular structures when added to water and, more particularly, to such compositions for use in treating dry skin conditions.

Emollient oils are routinely used to lubricate dry skin, for example in patients suffering from eczema, and which can extend over the entire body surface. Rather than annoint the body in oils, it is usually more convenient to add oil to a bath and in which the patient is then immersed. This has the advantage of also removing shed skin prior to the body being covered with oil.

The oils conventionally employed in such oil baths are usually blends of vegetable oils comprising triglycerides, mineral oils and esters of fatty acids commonly known as fixed oils. The type of oil is not considered to be as important as its cosmetic and/or therapeutic properties. To cater for personal preferences, bath oils may either float or disperse in the bath water. Floating bath oils are simple blends of oils which spread on top of the bath. They do not contain surfactants or emulsifying agents. This type is sometimes preferred because the spreading film of oil is perceived to cover the body more efficiently.

Alternatively, surfactants or emulsifying agents may be included with the oil so that it disperses into small oil globules when added to water. This type of bath oil is usually regarded as being more cosmetically acceptable. However, the high levels of surfactants required to disperse and emulsify the oil in the bath water can be irritant and sensitising to individuals who have sensitive skin. Furthermore, the surfactants commonly used are ethoxylated compounds which give rise to environmental problems during both their manufacture and eventual disposal. In particular, the method of manufacture involves solvents which may adversely affect the environment. It is also believed that ethoxylated surfactants can delay the skin's healing process, and this is obviously an undesirable effect for a bath oil since sufferers of dry skin conditions are particularly prone to cuts and abrasions.

The need for skin-hydration is another factor that is frequently mentioned when treating dry skin conditions. In practice, this is achieved by the use of moisturising agents to maintain a high relative humidity in the micro environment immediately above the epidermis. It is therefore desirable to include moisturisers in bath and other skin care products. However, the compounds that act as moisturisers are mostly water soluble and thus present considerable formulation problems in compositions containing oils. Furthermore, large amounts would have to be used to compensate for dilution of the bath oil in the bath.

Liposomes are lipid vesicles made up of membrane lipids arranged as alternating bilayers separated by aqueous spaces. Due to their ability to trap water in the aqueous spaces, liposomes are known to be ideal moisturisers. Above the phase transition temperature (T_{c}), membrane lipids preferentially organise into bilayer assemblies due to their amphipathic nature. Examples of naturally occurring membrane lipids are phospholipids and glycolipids. These may be unsaturated, partially saturated or fully saturated. The definition of phospholipids also covers enzyme hydrolysed phospholipids (i.e. lyso phospholipids), as well as chemically modified phospholipids (for example, hydroxylated and ethoxylated phospholipids). Examples of synthetic lipids which have bilayer forming properties include but are not limited to long chain dialkyl dimethyl ammonium compounds, mono and dialkyl polyoxyethylene derivatives and polyglycerol esters.

Lecithins are complex mixtures of phospholipids comprising chiefly phosphatidyl choline (PC), phosphatidyl ethanolamine (PE), phosphatidyl serine (PS) and phosphatidyl inositol (PI), together with varying contents of the equivalent lyso phospholipids and small amounts of other substances such as triglycerides, glycolipids, fatty acids and carbohydrates. Thus, lecithin is a generic name for a mixture of lipids and does not refer to a single type or blend of phospholipid. The degree of purity of a lecithin is usually determined by reference to the percentage of PC present. Pure grades of lecithin are generally regarded as containing at least 90% PC (expressed as a percentage of the total phospholipid content), while cruder grades of lecithin (as used in confectionery, for example) may contain only about 10% PC. Pure grades of lecithin are generally preferred in the preparation of liposomes used to carry biologically active compounds for dermatological applications. It is important in liposome work to define the grade of lecithin used because the type and the mixture of phospholipid it contains affects the properties of the liposomes.

Attempts to incorporate liposomes into oil-containing skin care products, particularly bath products, have generally not been successful. This is believed to be because the oil disrupts liposome membranes in the presence of water and the lipid hydrates and preferentially covers (emulsifies) the oil droplets instead of organising into vesicular structures. For these reasons, despite their potential benefits, bath oil preparations that contain or form liposomes have not been widely reported.

EP-B-0158441 describes "pro-liposome" compositions (i.e. compositions that are progenitors of liposomes) which comprise a uniform mixture of a) at least one membrane lipid and b) at least one water-miscible organic liquid that is a solvent for the lipid, and which spontaneously form liposomes upon coming into contact with excess water. Methods of forming liposomes on dilution of the pro-liposomes with excess water are also disclosed. While it has since been found that these pro-liposome compositions are generally quite suitable for inclusion in oils to form compositions that spontaneously form liposomes on dilution with water, the stability of the diluted suspension is not entirely satisfactory.

DE-A-4021083 purports to describe oil-containing liposome forming bath compositions for cosmetic and medicinal use. The compositions essentially contain pure lecithin (with a PC content of at least 90%) dissolved in a hydrophilic medium (e.g. ethanol) and one or more oils. They can thus be regarded as pro-liposome compositions formulated together with oils, and in consequence form liposomes upon addition of water. However, the requirement to use pure lecithin is a significant disadvantage since it is a very expensive component. While it is true that some brands of cosmetics can command premium prices, the inclusion of lecithin with a PC content of at least 90% means that the compositions are too expensive to achieve widespread use.

EP-A-0557825 claims oil bath and shower preparations with vesicle forming properties characterised by the presence of one or more surfactants together with one or more vesicle forming lipids. It is instructive to note that oil soluble polyoxyethylene derivatives are essentially present when forming vesicular structures according to this disclosure. Examples of surfactants are given as polyoxyethylene ethers and esters of fatty acids with HLB values between 6 to 13. The range of oil soluble surfactant content is said to be between 2% to 50%, especially from 10% to 20%. The preferred proportion of vesicle forming lipid is less than the surfactants and stated to be between 0.5% to 5%. It is apparent that relatively large amounts of ethoxylated surfactants are necessary to solubilise the lipid so that it can form vesicles and to disperse the oil. The accompanying photomicrographs claim to show vesicular structures. However, since they are not freeze fracture electron micrographs, it cannot be certain if they show liposomes or possibly emulsified oil droplets because of the high level of surfactant present. Furthermore, for the reasons noted above the inclusion of ethoxylated surfactants in bath preparations is undesirable.

There is therefore a need to have bath oils and other such preparations that do not contain harmful surfactants, but which nevertheless have good dispersion properties and are available at a reasonable price. They should also have good skin affinity and remain on the skin to lubricate and protect against trans epidermal water loss. A combination of these properties is often effective in alleviating dry skin conditions. It is an object of the present invention to provide such products and it has surprisingly been found that this can be achieved using a particular blend of phospholipids.

According to the present invention there is provided a composition comprising:-
i) a mixture of phospholipids in which phosphatidyl choline is present in an amount of from about 10% to about 80% and lyso phospholipid and/or chemically modified phospholipid in an amount of up to about 50%, as a percentage of the total lipid content of the said mixture, and
ii) one or more oils, at least one of which is a solvent for the phospholipids,
and which, in the substantial absence of ethoxylated surfactants and other oil-soluble surfactants, disperses upon coming into contact with water to form liposomes or vesicular structures and oil globules.

According to the present invention there is further provided a method of preparing a dispersion of liposomes or vesicular structures and oil globules in water, which comprises bringing the aforesaid composition into contact with water. The present invention further provides the dispersions of liposomes or vesicular structures formed from the aforesaid compositions and by the aforesaid method.

This invention thus provides oil-containing compositions, suitable for use as bath or shower preparations or for skin creams or lotions for example, which form liposomes or vesicular structures when added to, or otherwise placed in contact with, water or other aqueous media. They do not contain any harmful or membrane perturbing synthetic surfactants and, in consequence, are gentle and non-irritating and so are suitable for regular use to treat dry skin conditions. They are effective in moisturising and re-hydrating the skin. It has now been found that the mixtures of phospholipids used in this invention can be dissolved in vegetable, mineral and/or synthetic oils to give compositions that form separate and stable populations of discrete liposomes and oil droplets or globules when added to water. Unlike previous teachings, the compositions do not contain surfactants, are homogeneous and in fact are indistinguishable from ordinary oils. They do not involve the use of large amounts of expensive pure lecithin. However, they disperse readily in water and form liposomes spontaneously.

This surprising and advantageous behaviour is attributed to the unique mixture of phospholipids and the concentrations thereof that are employed. The combination of lyso phospholipid, preferably lyso phosphatidyl choline (LPC), and PC in the mixture unexpectedly increases the dispersibility of the oil component. Although it cannot be certain, LPC probably inserts between PC molecules and, depending on the molar ratios, either forms smaller liposomes or mixed micelles. In any event, the stability of the resulting suspension comprising liposomes and oil-droplets is much improved. It may also be that the oil used has an important role. Accordingly, the phospholipids for use in the compositions of this invention are special blends of lecithins which contain 10% to 80% of PC and up to about 50% lyso phospholipid and/or chemically modified phospholipid, expressed as a percentage of the total lipid content. The balance may comprise smaller amounts of the phospholipids PE and/or PS and/or PI, as well as non-phospholipid lipid components such as glycolipids, triglycerides and free fatty acids.

The lyso phospholipid component of the blend comprises the lyso equivalents of PC, PE, PS and/or PI. LPC usually forms the major fraction, followed by LPE. While their use is not preferred, it is nevertheless possible to have present chemically modified phospholipids, eg acetylated or hydroxylated phospholipid, in addition to or in place of lyso phospholipid. These are obtained, for example, by acetylating the PE component or hydroxylating across double bonds and should be familiar to those skilled in the art.

Examples of phospholipid blends suitable for use in this invention are the commercial lecithin compositions marketed by Lucas Meyer under the names PRO-LIPO E and EPIKURON E (Trade Marks). It should be understood that while the PRO-LIPO E and EPIKURON E products are commercially available blends, other mixtures of phospholipids or lecithin can of course be used. However, the phospholipid mixture should be composed of substantially alcohol soluble fractions comprising from about 10% to about 80% of PC, more preferably between about 20% to about 50% PC. The lyso phospholipids and/or modified phospholipids should be present in an amount of up to about 50%, typically from about 1% to about 50% and most preferably, in terms of LPC content, from about 5% to about 25%. Preferably, the ratio of LPC and/or chemically modified phospholipid to PC in the composition is from 1:50 to 1:1, more preferably from 1:20 to 1:2. If too little LPC is present the dispersibility of the oil composition may not be satisfactory. If too much is present it tends to separate out and causes stability problems. Therefore the correct balance has to be maintained to optimise the formulation. As a rule, higher levels of LPC in the mixture require correspondingly higher amounts of PC.

The concentration of total phospholipid present is generally between 0.5% to 10%, preferably about 1% to 5%. Below about 0.5% phospholipid content, very few liposomes are seen. Above about 10%, the phospholipids in the composition may separate or aggregate and may not disperse easily in water.

Optionally, the phospholipids may be dissolved or dispersed in a hydrophilic medium, such as methanol, ethanol, n-propyl alcohol, isopropyl alcohol and, where appropriate, glycerol, propylene glycol or mixtures thereof.

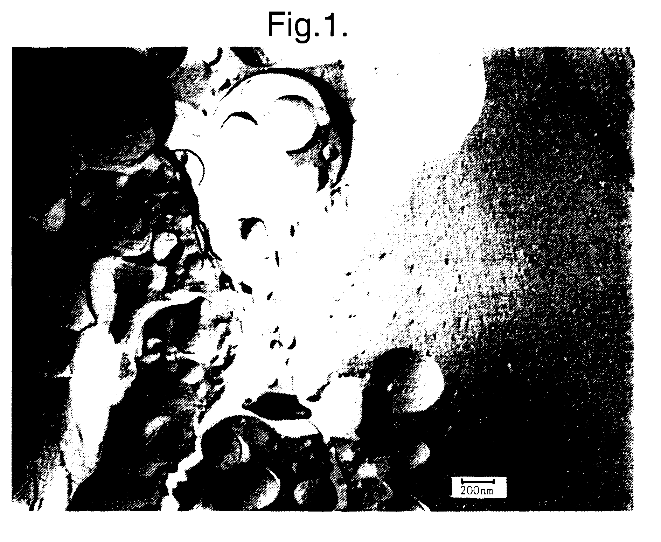

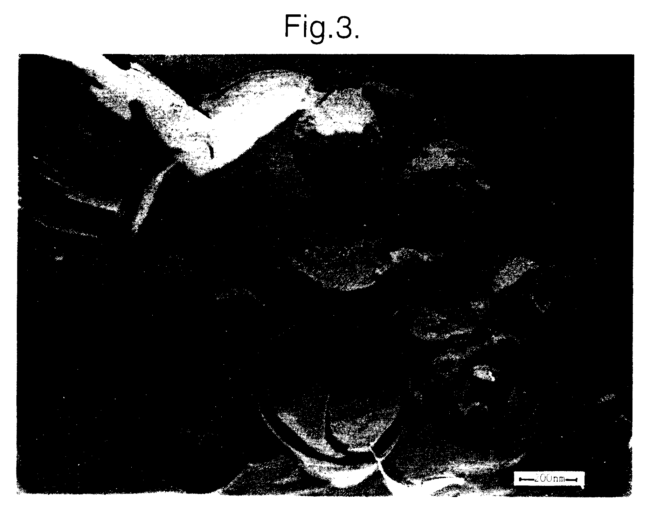

Although single oils can be used in the compositions, it is best to use blends of at least two. Vegetable oils e.g. soyabean, sunflower, groundnut, almond and olive oils are all suitable. Paraffins, fatty acid esters and cyclic hydrocarbons can also be included. At least one of the components should be a solvent for the lipid without affecting liposome formation. The preferred oils in this respect are branched chain alcohols/acids, preferably between C₁₂ to C₂₀, and the corresponding branched and straight chain mono and dibasic esters. Some oils such as isopropyl myristate may disrupt liposomes and are not favoured and so should not be used in large amounts. There is no hard and fast rule regarding the type of oil that may be used, as long as it is compatible and allows liposomes and oil globules to form spontaneously and exist as stable and separate populations. To meet this proviso, it is of the utmost importance that negative stain and freeze fracture electron microscopy and other appropriate techniques, when available, be used to validate liposome structure. Typical freeze fracture micrographs taken of Examples 1 and 4 below are shown in Figures 1 and 3.

It should be appreciated that biologically active compounds may be included in the composition so that, on conversion to liposomes, they become entrapped in the liposomes. Biologically active compounds include but are not limited to vitamins, antimicrobials, moisturisers, lipophilic compounds, emollients and fragrances. Examples of vitamins include vitamins A, C, D and E. Examples of antimicrobials are Triclosan (Trade Mark), Euxyl (Trade Mark) and phenoxyethanol, while glycerol and other polyols illustrate moisturisers that may be carried. In addition, compounds conventionally employed as anti-oxidants, preservatives and fragrances may also be included in the oil composition.

The following are typical examples of liposome forming oil compositions according to this invention:-

### Example 1

| | |
|---|---|
| *Phospholipid blend | 2% |
| Light liquid paraffin | 30% |
| Squalane | 30% |
| Branched chain C₁₆ alcohol | 38% |

| | |
|---|---|
| *Ethanol soluble fraction with approximately 45% PC and 10% LPC | |

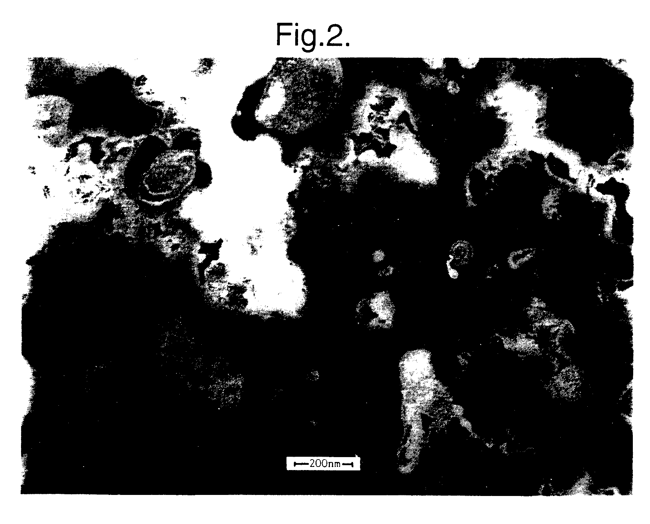

The phospholipid mixture was dissolved in the C₁₆ alcohol. The light liquid paraffin and squalane were added to obtain a homogeneous solution. 1 gm of this preparation was added to 100 ml of tap water at about 45°C and stirred lightly to form a milky suspension. The freeze fracture and negative stain electromicrographs of the liposome suspension which also contained oil droplets are shown in Figure 1 and Figure 2, respectively.

### Example 2

| | |
|---|---|
| *Phospholipid blend | 5% |
| Ethanol | 2.5% |
| Lauryl ester | 10% |
| Soya oil | 82.5% |

| | |
|---|---|
| *40% PC and 15% LPC | |

### Example 3

| | |
|---|---|
| *Phospholipid blend | 7.5% |
| Myristyl ester | 10% |
| Isopropyl palmitate | 5% |
| Sunflower oil | 77.5% |

| | |
|---|---|
| *50% PC and 2.5% LPC | |

### Example 4

| | |
|---|---|
| *PRO-LIPO E | 10% |
| C₁₈ branched chain alcohol | 20% |
| Liquid paraffin | 35% |
| Ground nut oil | 35% |

| | |
|---|---|
| *40% PC, 12% LPC, 4% LPE | |

All four components were blended together until homogeneous. The freeze fracture electronmicrograph of the sample prepared as in Example 1 is shown in Figure 3.

### Example 5

| | |
|---|---|
| *Phospholipid blend | 5% |
| Isopropyl alcohol | 5% |
| Cetyl lactate | 10% |
| Soya oil | 80% |

| | |
|---|---|
| *50% PC with 10% acetylated PE | |

### Example 6

| | |
|---|---|
| *Phospholipid blend | 2.5% |
| Ethanol | 2.5% |
| Isopropyl palmitate | 10% |
| Soya oil | 85% |

| | |
|---|---|
| *60% LPC and 20% PC | |

### Example 7

| | |
|---|---|
| *Phospholipid blend | 7.5% |
| Ethanol | 5% |
| Isopropyl myristate | 2.5% |
| Soya oil | 85% |

| | |
|---|---|
| *20% PC and 5% LPC | |

It should be noted that the special blends of phospholipids shown in these Examples are lecithin compositions containing the stated proportions of PC and LPC, in addition to the other lipid components which may be present in lecithins as described above. It is to be understood, however, that it is possible to use mixtures of pure PC and LPC or other modified phospholipids on their own.

The compositions from Examples 1 to 7 were further tested for dispersibility as follows. 0.25g of each formulation was added to 1 litre of hot tap water (45°C) contained in a one-litre pyrex beaker and stirred. A translucent dispersion was obtained. No clumping or agglomeration of the suspensions were seen even after standing for 12 hours.

This invention discloses homogeneous compositions comprising specified blends of substantially alcohol soluble phospholipids solubilised in fixed oils in the absence of ethoxylated surfactants and other oil soluble surfactants such that, on addition to water, they disperse spontaneously to form discrete populations of liposomes and oil globules. It is understood, however, that the disclosure is not restricted to bath oils. Indeed, it covers liposome forming oil compositions for all types of application externally and internally.

## Claims

1. A composition comprising:-
i) a mixture of phospholipids in which phosphatidyl choline is present in an amount of from about 10% to about 80% and lyso phospholipid and/or chemically modified phospholipid in an amount of up to about 50%, as a percentage of the total lipid content of the said mixture, and
ii) one or more oils, at least one of which is a solvent for the phospholipids,
and which, in the substantial absence of ethoxylated surfactants and other oil-soluble surfactants, disperses upon coming into contact with water to form liposomes or vesicular structures and oil globules.

2. A composition as claimed in claim 1, wherein component i) comprises lecithin.

3. A composition as claimed in claim 1 or claim 2, wherein component i) comprises phosphatidyl choline in an amount of from 20% to 50% and lyso phosphatidyl choline in an amount of from 5% to 25% as a percentage of the total lipid content.

4. A composition as claimed in any one of the preceding claims, wherein the lyso phospholipid comprises lyso phosphatidyl choline and the ratio of lyso phosphatidyl choline to phosphatidyl choline is from 1:50 to 1:1, preferably from 1:20 to 1:2.

5. A composition as claimed in any one of the preceding claims, wherein the phospholipids are dissolved/dispersed in a hydrophilic medium.

6. A composition as claimed in any one of the preceding claims, which has a total lipid content of from 0.5% to 10% by weight and, preferably, from 1% to 5% by weight.

7. A composition as claimed in any one of the preceding claims, wherein component ii) comprises at least one vegetable oil, mineral oil or synthetic oil.

8. A composition as claimed in any one of the preceding claims, wherein component ii) comprises at least one C₁₂ to C₂₀ branched chain alcohol or acid, or the corresponding branched or straight chain mono or dibasic ester.

9. A composition as claimed in any one of the preceding claims, which includes a biologically active compound.

10. A composition as claimed in any one of the preceding claims, which is formulated for use as a bath oil.

11. A method of preparing a dispersion of liposomes or vesicular structures and oil globules in water, which comprises adding a composition as claimed in any one of the claims 1 to 10 to water.

## Patentansprüche

1. Eine Zusammensetzung bestehend aus:-
i) einer Phospholipidenmischung, in der Phosphatidylcholin zu einem Anteil zwischen circa 10% und circa 80% und Lysopholipid und/oder chemisch modifiziertes Phospholipid zu einem Anteil von circa 50% enthalten sind, und zwar als Prozentsatz des gesamten Lipidenanteils der genannten Mischung und
i) eine oder mehr Öle, wovon mindestens eins ein Lösungsmittel für die Phospholipiden ist
und die, wenn keine ätholinhaltigen oberflächenaktiven Substanzen und andere öllöslichen oberflächenaktiven Substanzen im wesentlichen vorhanden sind und in Kontakt mit Wasser kommen, sich feinverteilen, um Liposomen oder blasenähnliche Strukturen und Ölkügelchen zu bilden.

2. Eine Zusammensetzung laut Anspruch 1, wobei Bestandteil i) Lezithin enthält.

3. Eine Zusammensetzung laut Anspruch 1 oder Anspruch 2, wobei Bestandteil i) Phosphatidylcholin zu einem Anteil von 20% bis 50% und Lysophosphatidylcholin zu einem Anteil von 5% bis 25% als Prozentsatz des gesamten Lipidenanteils enthält.

4. Eine Zusammensetzung laut Anspruch eines der obigen Ansprüche, wobei das Lysospholipid Lysophosphatidylcholin enthält und das Verhältnis des Lysophosphatidylcholin zu Phosphatidylcholin zwischen 1:50 bis 1:1 beträgt, bevorzugt wird von 1:20 bis 1:2.

5. Eine Zusammensetzung laut Anspruch eines der obigen Ansprüche, wobei die Phospholipiden in einem wasseraufnehmenden Lösungsmittel aufgelöst /feinverteilt werden.

6. Eine Zusammensetzung laut Anspruch eines der obigen Ansprüche, die einen gesamten gewichtsmäßigen Lipidenanteil von 0,5% bis 10% hat, gewichtsmäßig von 1% bis 5% ist besser.

7. Eine Zusammensetzung laut Anspruch eines der obigen Ansprüche, wobei Anteil ii) mindestens ein Pflanzenöl, Mineralöl oder künstliches Öl enthält.

8. Eine Zusammensetzung laut Anspruch eines der obigen Ansprüche, wobei Anteil ii) mindestens einen C₁₂ bis C₂₀ verzweigtkettigen Alkohol oder Säure enthält oder den entsprechenden verzweigt- und geradkettigen Mono- oder zweibasigen Ester.

9. Eine Zusammensetzung laut Anspruch eines der obigen Ansprüche, die einen biologisch aktiven Bestandteil enthält.

10. Eine Zusammensetzung laut Anspruch eines der obigen Ansprüche, die so formuliert ist, daß sie für Badeöl geeignet ist.

11. Eine Methode für die Feinverteilung der Liposomen oder blasenähnlichen Strukturen und Ölkügelchen in Wasser, bei der es möglich ist, daß eine Zusammensetzung laut Anspruch eines der obigen Ansprüche 1 bis 10 zu Wasser gegeben werden kann.

## Revendications

1. Une préparation comprenant :-
i) Un mélange de phospholopides dans lequel est présent une quantité d'environ 10% à 80% de phosphatidyl choline et une quantité allant jusqu'à environ 50% de lyso phospholopides et/ou de phospholopides modifiés chimiquement, en un pourcentage du contenu total en lipide du dit mélange, et
ii) une ou plusieurs huiles, dont une au moins est un solvant des phospholopides,
et qui en absence notable de tensio-actifs éthoxylés et autres tensio-actifs solubles dans l'eau, se disperse au contact de l'eau pour former des liposomes ou des structures vésiculaires et des gouttes d'huile.

2. Une préparation comme affirmée dans la déclaration 1 dans laquelle le composant i) comprend de la lécithine.

3. Une préparation comme affirmé dans les déclaration 1 ou 2, dans laquelle le composant i) comprend une quantité de 20% à 50% de phosphatidyl choline et une quantité 5% à 25% de lyso phosphatidyl choline en un pourcentage du contenu total de lipide.

4. Une préparation comme affirmée dans n'importe quelle des déclarations précèdentes dans laquelle le lyso phospholipide comprend du lyso-phosphatidyl choline soit de 1:50 à 1:1, de préfèrence de 1:20 à 1:2.

5. Une préparation comme affirmée dans n'importe quelle des déclarations précédentes, dans laquelle les phospholopides sont dissous/dispersés dans un milieu hydrophile.

6. Une préparation comme affirmée dans n'importe quelle des déclarations précédentes, dont le contenu total de lipide est soit de 0.5% à 10% en poids et de préférence de1% à 5% en poids.

7. Une préparation comme affirmée dans n'importe quelle des déclarations précédentes, dans laquelle le composant ii) comprend au moins une huile végétale, minérale ou synthétique.

8. Une préparation comme affirmée dans n'importe quelle des déclarations précédentes, dans laquelle le composant ii) comprend au moins un alcool/acide à chaînes ramifiées entre C₁₂ et C₂₀, ou l'ester mono ou dibasique correspondant à chaînes droites ou ramifiées.

9. Une préparation comme affirmée dans n'importe quelle des déclarations précédentes, qui inclut un composant actif biologiquement.

10. Une préparation comme affirmée dans n'importe quelle des déclarations précédentes, qui est formulée pour être utilisée comme huile pour le bain.

11. Une méthode de préparation d'une dispersion de liposomes ou structures vésiculaires ou gouttes d'huile dans l'eau, qui consiste en une préparation comme affirmée dans n'importe quelle des déclarations 1 à 10 dans de l'eau.
